(19) Europäisches Patentamt / European Patent Office / Office européen des brevets

(11) **EP 3 147 025 B1**

(12) **EUROPEAN PATENT SPECIFICATION**

(45) Date of publication and mention
of the grant of the patent:
**23.08.2023 Bulletin 2023/34**

(21) Application number: **15187231.4**

(22) Date of filing: **28.09.2015**

(51) International Patent Classification (IPC):
**B01F 27/1125** *(2022.01)*  **B01F 27/1144** *(2022.01)*
**B01F 27/13** *(2022.01)*  **B01F 27/191** *(2022.01)*
**B01F 27/70** *(2022.01)*  **B01F 35/30** *(2022.01)*
**B01F 35/41** *(2022.01)*  **C12M 1/107** *(2006.01)*
**C12M 1/06** *(2006.01)*

(52) Cooperative Patent Classification (CPC):
**C12M 21/04; B01F 27/11252; B01F 27/1144;**
**B01F 27/13; B01F 27/191; B01F 27/70;**
**B01F 35/412; C12M 27/06;** B01F 2035/351;
B01F 2035/352

(54) **FERMENTERS AND TANKS FOR STORING SEWAGE WITH A MIXER/STIRRER**

BIOGASFERMENTER SOWIE LAGERTANKS FÜR ABWASSER MIT MISCHER/RÜHRER

FERMENTEURS ET RÉSERVOIRS DE STOCKAGE AVEC MÉLANGEUR/AGITATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**29.03.2017 Bulletin 2017/13**

(73) Proprietor: **Corradi & Ghisolfi Srl**
**26010 Corte de' Frati (CR) (IT)**

(72) Inventors:
• **CORRADI, Paolo**
 **I-26010 Corte de' Frati (CR) (IT)**
• **CORRADI, Bruno**
 **I-26100 Cremona (IT)**

(74) Representative: **Fezzardi, Antonio et al**
**Studio Ferrario Srl**
**Via Collina, 36**
**00187 Roma (IT)**

(56) References cited:
EP-A2- 2 377 600  DE-A1- 10 260 972
DE-U1-202006 004 982  DE-U1-202010 009 323
DE-U1-202011 052 408  US-A- 628 232
US-A- 3 432 109  US-A1- 2009 042 267

**Description**

The present invention basically regards a mixing plant that can be used for mixing mixtures of matrices of livestock effluents, of by-products of the agro-industrial sector, and of biomasses, typically used in anaerobic-digestion plants, for mixing in storage tanks for livestock effluents or depuration slurry with low percentages of dry substance (preferably, but not exclusively, in the region of 10 - 15%).

Known to the art are stirrers-oxygenators, in particular aimed at obtaining homogeneization of livestock waste, which are equipped with an end propeller and have different configurations that enable use thereof in different situations: buried tanks, screens, tanks covered with trapdoors, external storage tanks, biogas plants, and lagoons. In some cases, an oxygenator-aerator accessory is provided, which introduces air into the liquid mass with the aim of increasing the fertilizing power thereof and favouring its fermentation to inhibit formation of smells in the case of aerobic fermentation or to favour homogeneization of the matrices in the case of anaerobic fermentation and consequently without the presence of the aeration system. Typically, these apparatuses are moved by electric or hydraulic motors or else are connected to the power takeoff of a tractor.

Currently, two mixing techniques are commonly used:

- slow mixing with apparatuses acting as flow accelerators (homogeneous flow with high capacity and low speed);
- fast mixing with apparatuses acting as mixers (small and fast propeller with localized swirling flow at high speed)

A problem common to these systems for sewage mixing/stirring using normal submersible mixer and fast flow lies in the fact that fast turbulence is created in the working fluid in reduced operating spaces. Moreover, these known apparatuses are unable to move the mix of matrices in the digester so as to create therein a gradual and uniform mixture between the various density gradients, this necessarily entailing undesirable formation of discontinuous stratification and creation of heterogeneous areas in the lower and upper parts of the fermenter that do not enable correct management of the times of retention and do not guarantee the absence of non-perfectly digested matrices discharged by the post-fermenter. Moreover, since they work at a high speed, they present a high energy absorption for their operation and are subject to a high wear of the mechanical seals and the propellers, the wear being due to erosion by friction with the mixture to be moved.

Another disadvantage of these known mixers is represented by the fact that they do not perform an effective "crust-breaking" action and require a specific action aimed at breaking down the surface crust of dried matrices with the aim of getting the fragments of said crust to sink into the underlying part of the sewage so as to send them back into solution.

Known from DE10260972 is an apparatus for stirring liquid ordure and/or waste water in an accumulation tank, which comprises a container with a shaft that turns about an axis of rotation, bearings, a motor, and remixing blades. The shaft extends radially in the container, and the blades are arranged on the shaft laterally with respect to the axis of rotation. In DE10260972 the rotation shaft is fixed to the lid of the tank, and each blade is symmetrical with respect to the axis of rotation.

Also known, from DE202006004982, is a stirring mechanism for providing mechanical energy to fermentation tanks arranged vertically with at least one driving mechanism, a bearing for the stirring shaft that is attached to the side wall of the fermentation tank, a bearing for the stirring shaft that is located on the side where the fermentation tank is and rests on the bottom of the fermentation container by means of at least one support, and at least one stirring shaft with at least one stirrer blade that is fixed to the stirring shaft. The stirring shaft extends parallel to the bottom of the fermentation tank from the wall of the fermentation tank along the main axis of the fermentation tank. The shaft to which the blades of said stirrer are fixed is located at a distance from the bottom of the tank such that the ends of said blades skim the bottom of the tank itself and such that said blades are always entirely submerged in the liquid contained in the tank, or else the shaft to which said blades are fixed is located at a distance from the bottom of the tank such that the ends of said blades project from the free surface of the liquid but remain always very far away from the bottom thereof.

Moreover, DE202011052408 describes a stirrer, in particular for a fermenter of a biogas plant, with an axis of rotation on which various blades are arranged, each of which has at least one active surface, in which said surfaces are shaped and are arranged with respect to one another in such a way as to define the shape of a shaft with discontinuous screw. The blades of DE202011052408 are arranged at a certain distance from the axis of rotation and the stirrer is positioned in the top part of the fermenter, at a distance from the bottom.

The main purpose of the present invention is to overcome the problems outlined above by providing a plant with continuous or timed operation designed to maintain the composition of the sewage undergoing anaerobic digestion homogeneous and prevent formation of the surface crust, sending back into solution the materials that tend to form said crust.

The invention is defined by a fermenter/digester or a sewage-storage tank according to claim 1.

A better understanding of the invention will be obtained from the ensuing detailed description with reference to the attached drawings, which illustrate, purely by way of indication, some examples of the prior art and a preferred embodiment of the invention.

In the drawings:

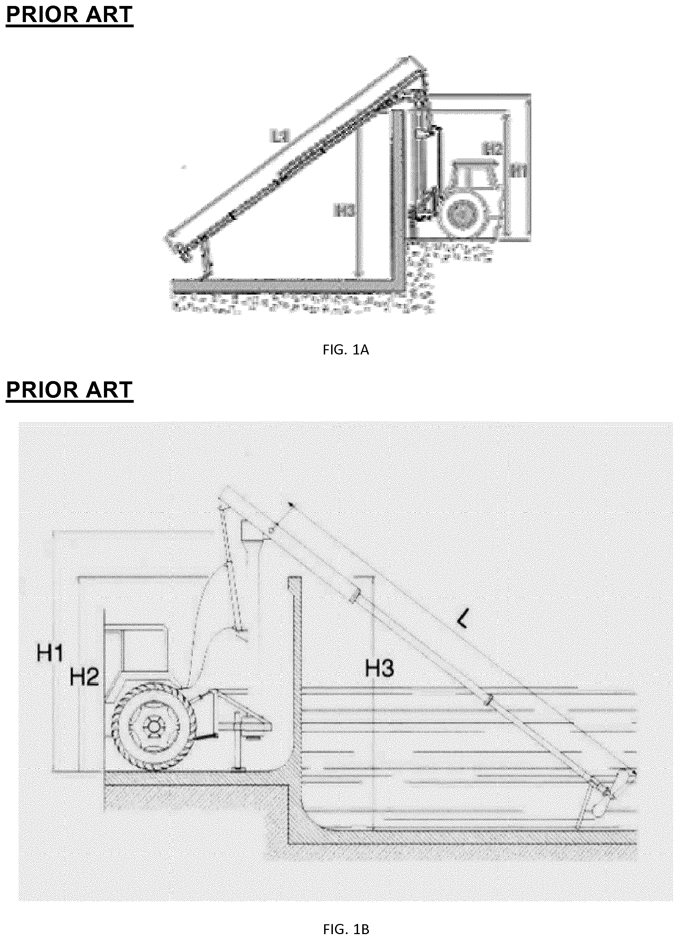

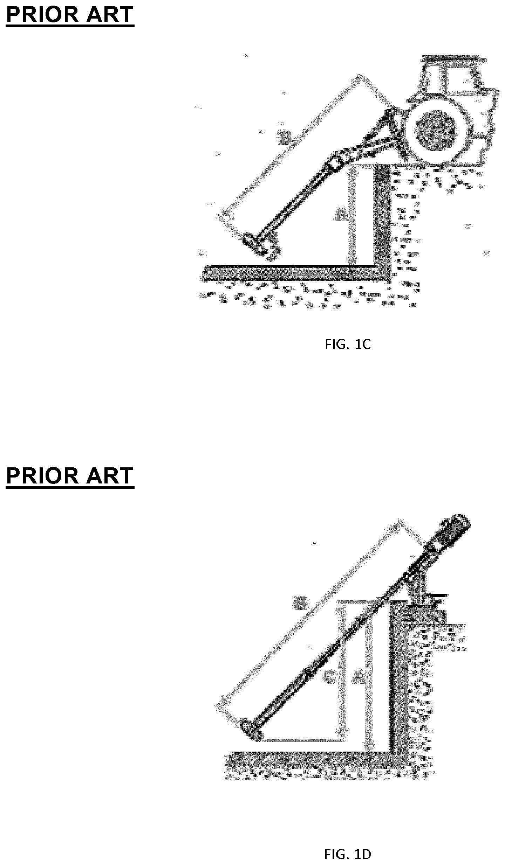

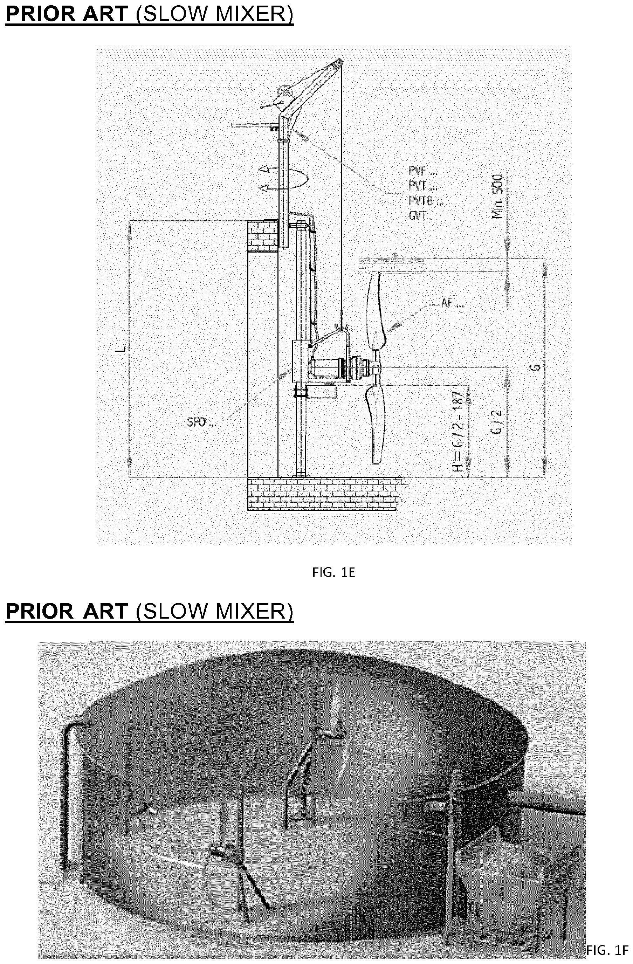

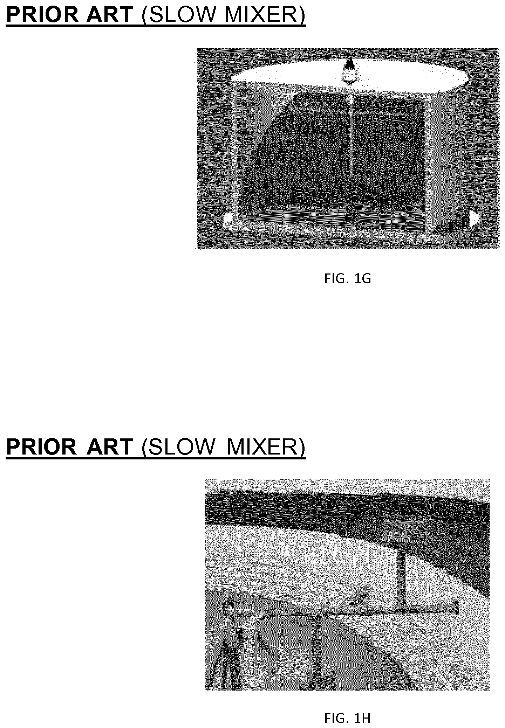

Figure 1A to 1H show some examples of the prior art;

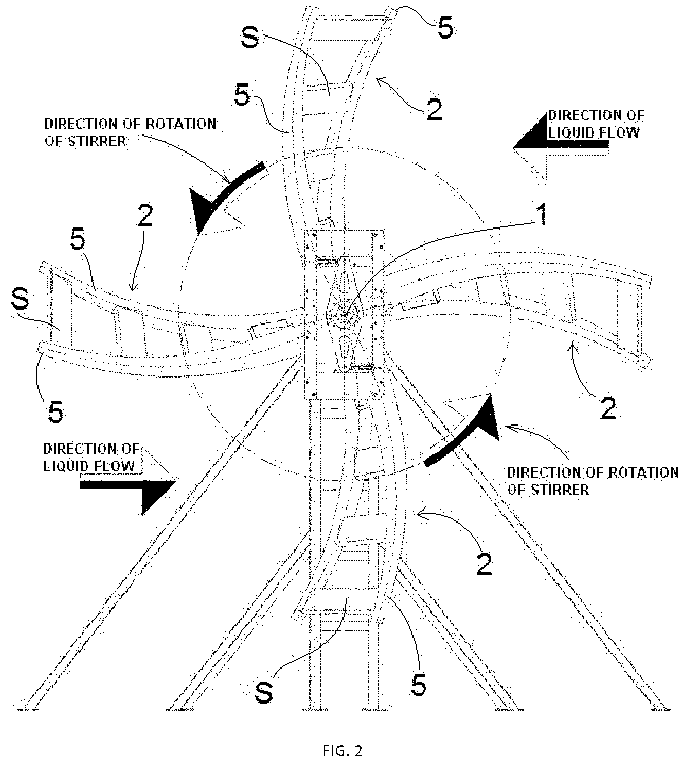

Figure 2 is a front view of the invention in which the direction of rotation of the blades is shown;

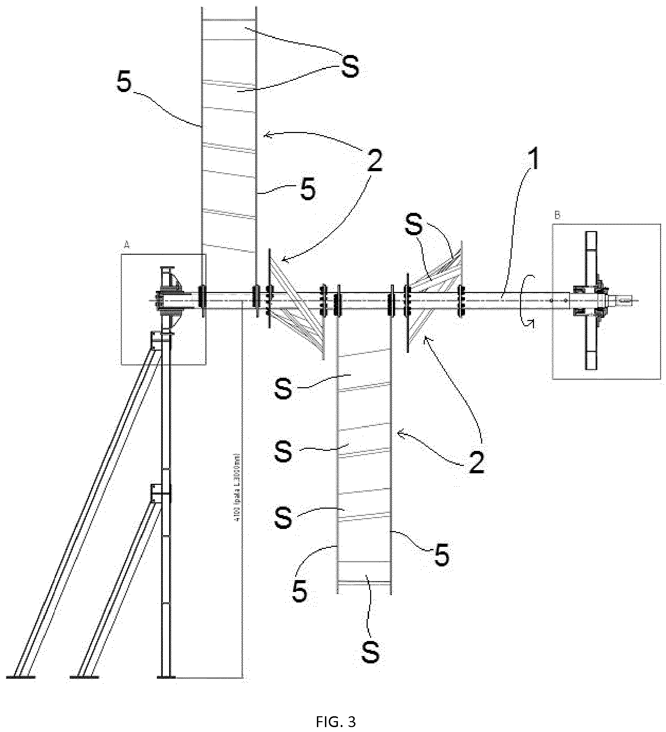

Figure 3 is a side view of the invention, corresponding to that of Figure 2;

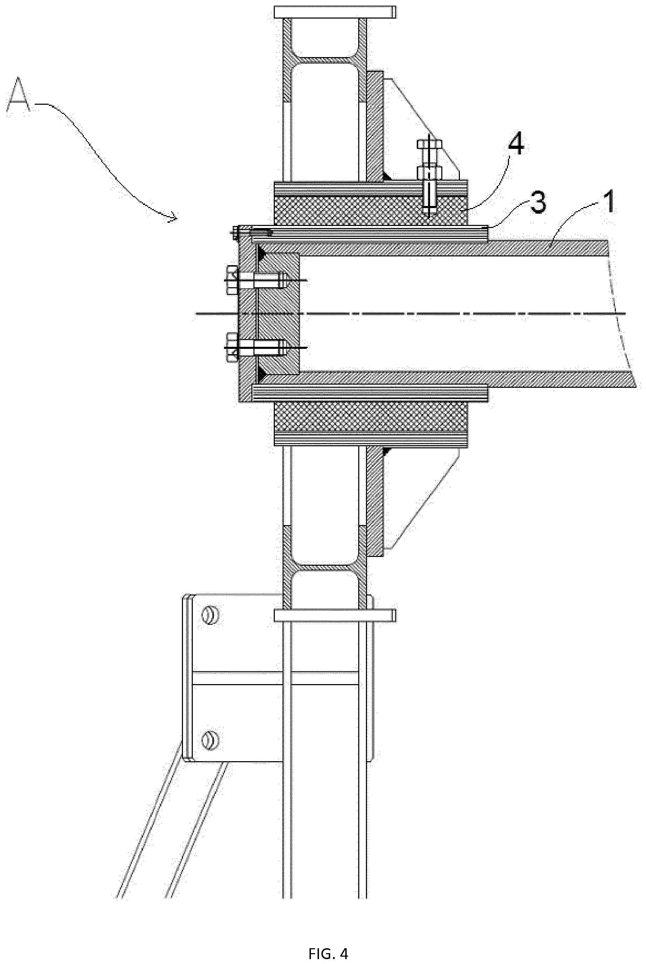

Figure 4 shows the constructional detail corresponding to the support internal to the digester, designated by "A" in Figure 3, partially sectioned;

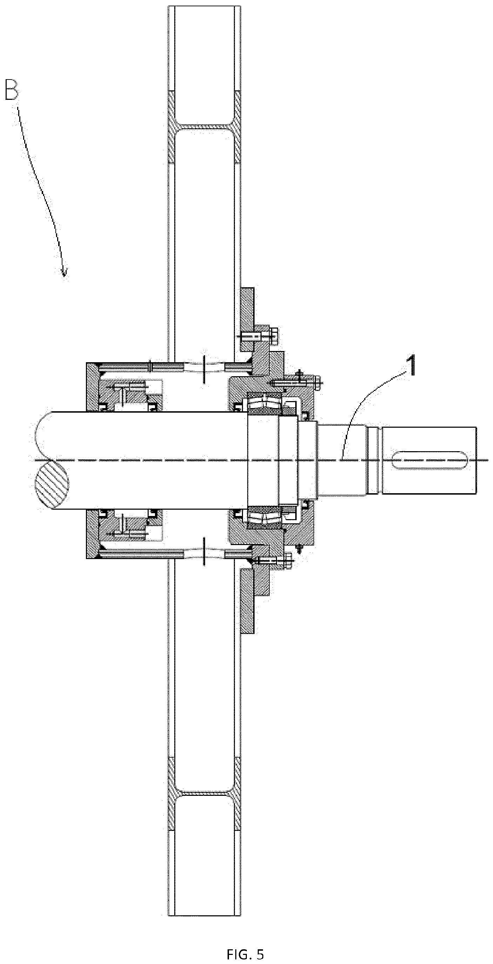

Figure 5 shows the constructional detail corresponding to the support on the wall of the digester, designated by "B" in Figure 3, partially sectioned;

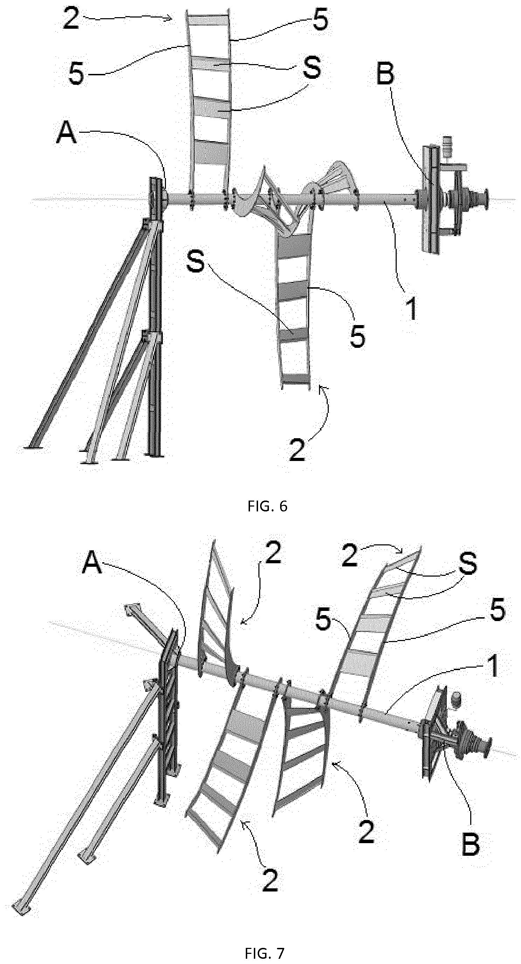

Figures 6 and 7 show 3D views of the embodiment illustrated in Figures 2 and 3;

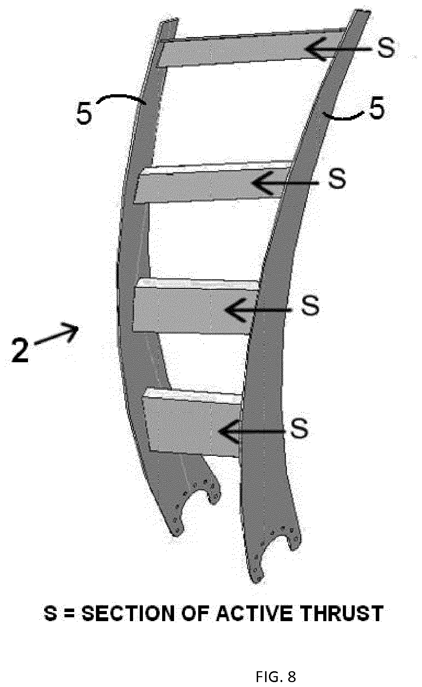

Figure 8 shows the main elements that constitute a blade according to the present invention; and

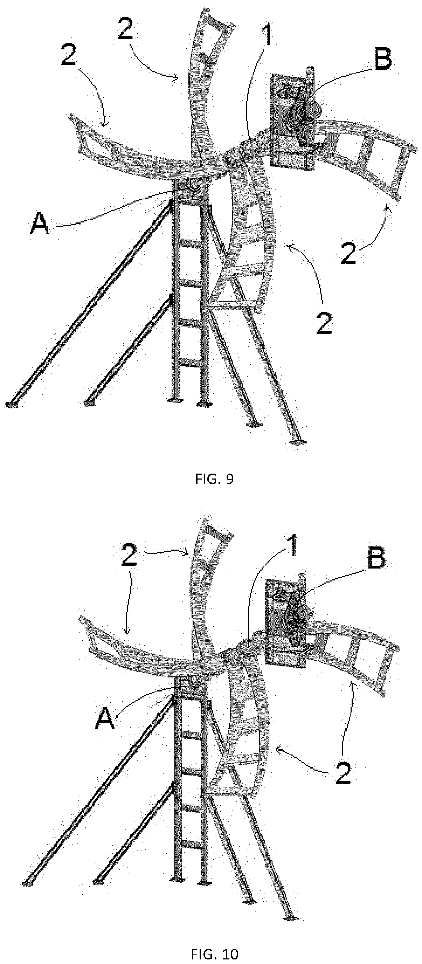

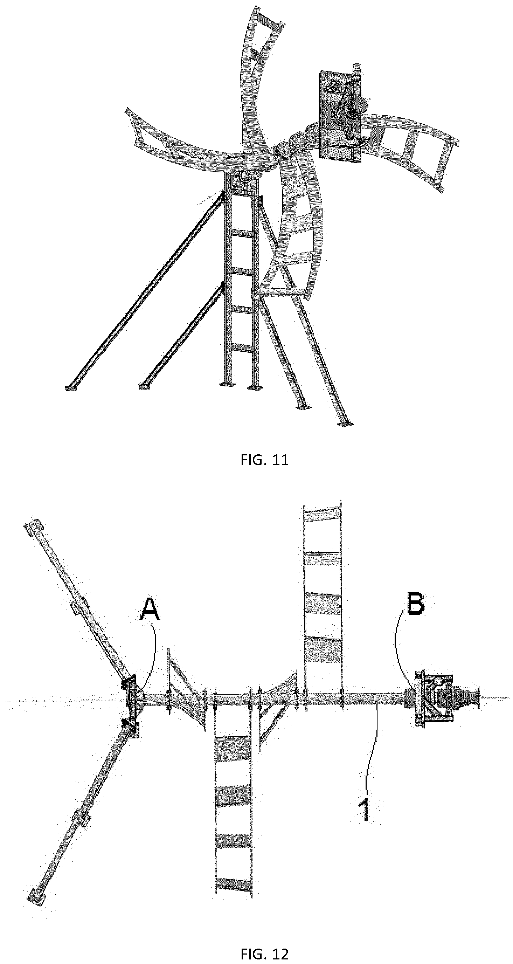

Figures 9 to 12 are other views that illustrate the configuration and conformation of the invention.

According to a first peculiar characteristic of the invention, the mixer has a horizontal axis of rotation 1 and is equipped with blades 2 with substantially radial development with respect to the axis of rotation of a shaft 1, said blades 2 being characterized in that they have a high hydrodynamicity and in that they have a shape curved like a spiral, as well as being fixed to the shaft 1 with an angular phase offset that renders them angularly equidistant from one another, which generates a thrust flow that is both axial and radial.

More specifically, the arms 5 of said blades 2 are warped and curved in the direction opposite to the direction of rotation.

The preferred embodiment that is described envisages four coaxial blades 2, regularly spaced apart along the axis of rotation, with an angular phase offset of 90° with respect to one another. Each blade 2 is set in cantilever fashion with respect to the shaft 1 on which it is fixed so as to project only from one side of the shaft itself.

In the case where the blades 2 were, for example, three, the angular phase offset would be 120° since they must be equidistant in order to guarantee a balanced rotation. Likewise, if there were five blades, the phase offset would be 72°, if there were six, it would be 60°. It could also function just with two blades set at 180°, even though perhaps less efficiently.

A second peculiar characteristic of the invention lies in the fact that said blades 2 are purposely sized in height to lap the free surface of the fluid in which they move, thus preventing and removing formation of any surface crust. Preferably, these blades 2 have a length such as to exit slightly from the surface of the mass to be mixed, even when this mass is at its maximum level.

In the example described purely by way of nonlimiting illustration, the mixer is driven by a motor (typically having a power of 15 kW) managed via inverter and is mounted on a reducer with a pre-set reduction ratio on the basis of the application (typically 142:1); in any case, the reduction ratio is chosen according to the stress caused by the hydraulic resistance of the fluid in which the mixer moves.

Typically, mixers work at a common peripheral velocity, according to the radius of the blade.

The mixer is preferably made of carbon steel or other material suited for the purpose.

A first support "A" - internal to the digester - is constituted by a chrome-plated sleeve 3 fitted on the shaft 1 and inserted in a bearing 4 made of wear-resistant polyzene, whereas a second support "B" - of a wall type - contains the roller bearing and a dual pressurized oil seal system.

According to another peculiar characteristic of the invention, the movement of the blades 2 and their inclination and curvature are such that the axial thrust is withstood by the support "B" on the wall of the digester.

The above choice derives from the need to reduce as much as possible any direct intervention on the blades or on the parts of the mixing system submerged in the fluid to be mixed and to isolate as much as possible the mechanical moving parts of the system from said fluid; moreover, this plant solution reduces the need for maintenance operations to be carried out inside the tank containing the fluid to be mixed.

The two outer seals of the wall support "B" can advantageously be taken down and replaced from outside without any need to operate in the fluid to be mixed.

The mixers can operate continuously or else in "work pause" mode. The motor can typically work at an operating frequency of 25-65 Hz: management of the mixing speed regulated by means of the inverter enables a regulation of the mixing speed to be obtained on the basis of the effective need of the bioreactor, thus leading to a saving in terms of energy and wear.

The mixer makes it possible to obtain an optimal mixing in so far as the four blades 2 of which it is made up follow one another in the rotation, with a phase offset, as already said, of 90° with respect to one another and are set at different and regular distances along the mixing shaft 1.

According to a further peculiar characteristic of the invention, the angular and axial distance envisaged between the blades 2 is such that the work generated by one blade does not come to brake or affect the work produced by the blade that precedes it.

Yet another peculiarity of the invention lies in the geometry and in the constructional details of the individual blade 2: as compared to existing slow-mixing blades, in fact, the surface of "passive" friction on the supporting arms 5 of the pushers "S" has been minimized, reducing the thickness thereof to the benefit of a greater energy available for the section of active thrust constituted by the surface of the pushers "S" themselves.

Moreover, the particular shape of the blade 2, warped according to a spiral and curved with the concavity facing the axial component of the direction of the flow of displacement of the liquid to be treated, is designed to push towards the centre of the bioreactor the mass of liquid to be displaced, directing it towards the next blade 2 and thus obtaining, thanks to the length of the blade itself, four main effects:

1. imparting on the fluid to be mixed an orderly and continuous movement that proceeds maintaining the flow created as linear as possible and preventing phenomena of turbulence (laminar flow);

2. lightening the work (and hence reducing the energy necessary) for the next blade in so far as there do not occur phenomena of "hydraulic friction" between the flow of the liquid pushed by one blade 2 and the flow of the liquid pushed by the next blade 2, thus exerting an action of relaunching on the flows generated by the previous blade;

3. generating a linear swirl that creates on the surface of the working fluid a circular movement that is well suited to the shape of the tanks and of the digesters with circular section;

4. lapping the free surface of the fluid, removing and preventing formation of crusts and accumulations of substances in suspension (surface crusts).

It should be noted that the differences of density and size between the particles that make up the mixture of the matrices that constitute the typical working fluid cause a slight stratification in terms of density gradient within the tank or digester in which they are contained: the organic solid matrices such as, for example, manure or silage, in fact, tend to stratify in the upper part of the digester, forming the so-called "cap", whilst the sewage and the organic substances that are in an advanced state of digestion will occupy progressively deeper parts of the digester itself.

The mixing system must take into account the fact that, in stationary conditions, the greater the height of the fermenter, the more marked the separation by density gradient of the matrices being digested, which will have a greater space for levelling in the digester on the basis of their density.

This effect causes a natural stratification of the matrices in the fermenters, and in particular, in the specific case, the matrices with greater organic load and lower density, with longer degradation times, which consequently require longer retention times, tend to occupy the upper part of the digester (or even to float on the surface of the working fluid), whereas the matrices that are more easily degradable, with lower organic content and higher density, and hence with the need for shorter retention times, such as sewage and matrices in advanced state of degradation, tend to occupy the lower part of the digester.

The mixing systems according to the present invention envisage blades 2 with wide radius having a substantially horizontal axis of rotation 1, which, unlike normal fast-flow submersible mixers, which create fast turbulence in the working fluid in reduced operating spaces, are designed to move the mix of matrices in the digester creating therein a uniform mixture between the various density gradients so as to eliminate discontinuous stratification and at the same time prevent creation of heterogeneous areas in the lower and upper parts of the fermenter. The presence of these heterogeneous areas, which are characteristic of currently known systems, does not enable correct management of the retention times and do not guarantee the absence of non-perfectly digested matrices discharged by the post-fermenter.

It should be noted that, according to a further peculiar characteristic of the invention, the crust-breaking effect of the blades that emerge from the surface of the fluid causing forced sinking of possible matrices in suspension enables recirculation of the organic matrices that tend to float, constantly sending them back into solution.

The above effect, which has been purposely studied and generated by the mixing systems according to the present invention, may be ideally represented by the fundamental equation that describes the speed of sedimentation of a particle in suspension, subjected to an acceleration

$$ v = \frac{2\, r_P^2 (\rho_P - \rho_M)\, \omega^2 r}{9\, \eta\, (f\,/\,f_0)} $$

where:

v is the terminal speed of the particle
$r_P$ is the radius of the particle
$\rho_P - \rho_M$ is difference between the density of the matrix being digested $\rho_P$ and the density of the mixture in which they are suspended $\rho_M$;
$\omega$ is the angular velocity of the mixture being digested (in radians per second);
r is the distance between the particle and the axis of rotation;
$\eta$ is the coefficient of viscosity of the medium;
$f/f_0$ is the ratio of friction, i.e., the ratio between the hypothesized coefficient of friction f of the 'real' matrix and the coefficient of friction $f_0$ for a perfectly spherical and non-hydrated particle (in practice, it is a correction factor that takes into account the different shape and different surface characteristics of the particles).

From the above equation it is evident that the speed at which the matrix moves in the mixture, tending to drop into the lower part, basically depends upon the dimensions (radius squared) and upon the density of the particle itself: hence, generally, matrices that are more solid and larger but less dense will tend to move at a moderate speed and will gradually reach the mean height of the reactor, at the same time losing dry substance with the rise in density and the reduction in size as a result of degradation, thus creating a gradient that is progressively homogeneous along the height of the fermenter.

Since the matrices and substances in a homogenized such as the material being digested have different sizes, densities, and shapes linked to their state of degradation (the smaller size, the greater the ease of degradation), they will be separated - also thanks to the movement of the blades - on the basis of their different sedimentation rate that varies in the course of the process, thus enabling efficient mixing thereof.

"Combi" mixers have been designed and made in compliance with the Machine Directive 2006/42/CE.

It should be noted that the dimensional ratio between the size of the blades 2 and their radius of curvature is the result of experiments, whereas the ratio between their length and their width depends principally upon the power of the motor.

## Claims

1. A fermenter/digester or a sewage-storage tank having a maximum level of filling, said fermenter/digester or sewage-storage tank comprising a mixer/stirrer provided with a horizontal-axis rotary shaft (1), said shaft (1) being equipped with blades (2), which are mounted in cantilever fashion and have a substantially radial development with respect to the rotary shaft (1), said blades (2) being mutually staggered and being set apart from one another along the shaft (1) and being fixed thereto with an angular offset that renders them angularly equidistant from one another and being warped and curved so as to generate a thrust flow that is both axial and radial;

   wherein said blades (2) are configured to have a length such that, even when fluid to be treated in which they move is at the maximum level of filling envisaged by said fermenters/digesters and sewage-storage tanks, the free ends of said blades (2) are configured to exceed said maximum level so as to lap the free surface of the fluid to be treated in order to prevent formation of surface crusts; and
   wherein said blades (2) have a wide radius and a substantially horizontal axis of rotation, so as to generate a uniform mixture in said fermenters/digesters and sewage-storage tanks between different density gradients, thereby eliminating discontinuous stratification and preventing creation of heterogeneous areas at lower and upper parts of said fermenters/digesters and sewage-storage tanks,
   wherein
   said blades (2) have a shape curved like a spiral and are warped and curved in a direction opposite to the direction of rotation; wherein said mixer/stirrer envisages a plurality of coaxial blades (2), regularly spaced apart along the axis of rotation, with an angular phase offset that renders them angularly equidistant from one another.

2. The fermenter/digester or sewage-storage tank according to Claim 1, **characterized in that** said mixer/stirrer envisages four coaxial blades (2), regularly spaced apart along the axis of rotation, with an angular offset of 90° with respect to one another.

3. The fermenter/digester or sewage-storage tank according to any one of the preceding claims, **characterized in that** said blades (2), which have a length such as to emerge slightly from the surface of the fluid mass to be mixed, are designed to provide a "crust-breaking" effect, causing forced sinking of possible matrices in suspension, thus enabling recirculation of the organic matrices that tend to float, bringing them constantly into solution.

4. The fermenter/digester or sewage-storage tank according to any one of the preceding claims, **characterized in that** said mixer/stirrer is made of carbon steel or other material suited for the purpose.

5. The fermenter/digester or sewage-storage tank according to any one of the preceding claims, **characterized in that** said mixer/stirrer envisages a first support (A) within the digester constituted by a chrome-plated sleeve (3) fitted on the shaft (1) inserted in a bearing (4) made of wear-resistant polyzene, as well as a second support (B) shaped like a wall that contains a roller bearing and a dual pressurized oil seal system, for isolating as much as possible the mechanical moving parts of the system from the working fluid.

6. The fermenter/digester or sewage-storage tank according to Claim 5, **characterized in that** the two outer seals of the second support (B) can be taken down and replaced from outside without any need to operate in the fluid to be mixed.

7. The fermenter/digester or sewage-storage tank according to any one of the preceding claims, **characterized in that** said mixer/stirrer is equipped with driving means for enabling continuous operation or else operation in "pause" mode.

8. The fermenter/digester or sewage-storage tank according to Claim 7, **characterized in that** said mixer/stirrer envisages means for driving the motor to adjust the mixing speed by means of a purposely provided inverter in order to obtain adjustment of the mixing speed on the basis of the effective need of the bioreactor, thus leading to a saving in terms of energy and wear.

**9.** The fermenter/digester or sewage-storage tank according to Claim 2, **characterized in that** the angular and axial distance envisaged between the blades (2) is such that the work generated by one blade (2) does not come to brake or affect the work produced by the blade (2) that precedes/follows it.

**10.** The fermenter/digester or sewage-storage tank according to Claim 1, **characterized in that** the length and the particular shape of each blade (2), which is warped according to a spiral and curved with concavity facing the axial component of the direction of the flow of displacement of the liquid to be treated, is designed to push towards the centre of the bioreactor the mass of liquid to be treated, directing it towards the next blade (2), and thus obtaining that there is imparted on said liquid mass:

- a horizontal rotary movement within the bioreactor;
- a movement of horizontal displacement from the periphery towards the centre of the bioreactor; as well as
- a movement of mixing in the vertical direction that mixes the lighter matrices that tend to rise with the heavier ones that tend to sink in order to homogenize the mass to be treated; and
- a lapping of the free surface of the fluid, thus removing and preventing formation of surface crusts and accumulations of substances in suspension.

**11.** The fermenter/digester or sewage-storage tank according to any one of the preceding claims, **characterized in that** each blade (2) envisages a surface of "passive" friction on the supporting arms (5) of the pushers (S) that is minimized as compared to existing slow-mixing blades, reducing the thickness thereof to the advantage of a greater energy available for the active thrust section constituted by the surface of the pushers (S) themselves.

## Patentansprüche

**1.** Fermenter/Faulbehälter oder Abwasserspeicher mit einem maximalen Füllstand, wobei der Fermenter/Faulbehälter oder Abwasserspeicher einen Mischer/Rührer umfasst, der mit einer Drehwelle (1) mit horizontaler Achse versehen ist, wobei die Welle (1) mit Schaufeln (2) ausgestattet ist, die freitragend montiert sind und eine im Wesentlichen radiale Entwicklung in Bezug auf die Drehwelle (1) aufweisen, wobei die Schaufeln (2) zueinander versetzt und entlang der Welle (1) voneinander mit einem Abstand befestigt sind, der sie winkelmäßig äquidistant zueinander macht, und gekrümmt und gebogen sind, sodass sie einen sowohl axialen als auch radialen Schubfluss erzeugen;

wobei die Schaufeln (2) so konfiguriert sind, dass sie eine solche Länge haben, dass, selbst wenn das zu behandelnde Fluid, in dem sie sich bewegen, den maximalen Füllstand hat, der von den Fermentern/Faulbehältern und Abwasserspeichertanks vorgesehen ist, die freien Enden der Schaufeln (2) so ausgelegt sind, dass sie diesen maximalen Füllstand überschreiten, sodass sie die freie Oberfläche des zu behandelnden Fluids durchbrechen, um die Bildung von Oberflächenkrusten zu verhindern; und wobei die Schaufeln (2) einen großen Radius und eine im Wesentlichen horizontale Drehachse haben, sodass in den Fermentern/Faulbehältern und Abwasserspeichertanks ein homogenes Gemisch zwischen verschiedenen Dichtegradienten erzeugt wird, wodurch eine diskontinuierliche Schichtenbildung beseitigt und die Bildung heterogener Bereiche im unteren und oberen Teil der Fermenter/Faulbehälter und Abwasserspeichertanks verhindert wird,

wobei
die Schaufeln (2) eine spiralförmig gekrümmte Form haben und in einer Richtung entgegengesetzt zur Drehrichtung gekrümmt sind; wobei der Mischer/Rührer eine Vielzahl von koaxialen Schaufeln (2) vorsieht, die entlang der Drehachse regelmäßig mit Abstand voneinander angeordnet sind, mit einem winkelmäßigen Phasenversatz, der sie winkelmäßig äquidistant zueinander macht.

**2.** Fermenter/Faulbehälter oder Abwasserspeicher nach Anspruch 1, **dadurch gekennzeichnet, dass** der Mischer/Rührer vier koaxiale Schaufeln (2) vorsieht, die entlang der Drehachse regelmäßig mit Abstand voneinander angeordnet sind und einen Winkelversatz von 90° zueinander aufweisen.

**3.** Fermenter/Faulbehälter oder Abwasserspeicher nach einem der vorhergehenden Ansprüche, **dadurch gekennzeichnet, dass** die Schaufeln (2), die so lang sind, dass sie etwas aus der Oberfläche der zu mischenden flüssigen Masse herausragen, so konzipiert sind, dass sie einen "Krustenbrecher"-Effekt bewirken, der ein erzwungenes Absinken möglicher Matrizen in Suspension bewirkt und so eine Rückführung der organischen Grundmassen, die zum Aufschwimmen neigen, ermöglicht, wodurch sie ständig in Lösung gebracht werden.

**4.** Fermenter/Faulbehälter oder Abwasserspeicher nach einem der vorhergehenden Ansprüche, **dadurch gekennzeichnet, dass** der Mischer/Rührer aus Kohlenstoffstahl oder einem anderen für diesen Zweck geeigneten Material hergestellt ist.

**5.** Fermenter/Faulbehälter oder Abwasserspeicher nach einem der vorhergehenden Ansprüche, **dadurch gekennzeichnet, dass** der Mischer/Rührer eine erste Halterung (A) im Inneren des Faulbehälters vorsieht, die aus einer verchromten Hülse (3) besteht, die auf der Welle (1) angebracht ist, die in ein Lager (4) aus verschleißfestem Polyzen eingesetzt ist, sowie eine zweite Halterung (B), die wie eine Wand geformt ist, die ein Rollenlager und ein doppeltes Drucköl-Dichtungssystem enthält, um die mechanisch beweglichen Teile des Systems so weit wie möglich von der Arbeitsflüssigkeit zu isolieren.

**6.** Fermenter/Faulbehälter oder Abwasserspeicher nach Anspruch 5, **dadurch gekennzeichnet, dass** die beiden äußeren Dichtungen der zweiten Halterung (B) von außen abgenommen und ausgetauscht werden können, ohne dass in der zu mischenden Flüssigkeit gearbeitet werden muss.

**7.** Fermenter/Faulbehälter oder Abwasserspeicher nach einem der vorhergehenden Ansprüche, **dadurch gekennzeichnet, dass** der Mischer / Rührer mit Antriebsmitteln ausgestattet ist, die einen kontinuierlichen Betrieb oder einen Betrieb im "Pausenmodus" ermöglichen.

**8.** Fermenter/Faulbehälter oder Abwasserspeicher nach Anspruch 7, **dadurch gekennzeichnet, dass** der Mischer/Rührer Mittel zum Antrieb des Motors zur Einstellung der Mischgeschwindigkeit mittels eines eigens dafür vorgesehenen Inverters vorsieht, um eine Einstellung der Mischgeschwindigkeit auf der Grundlage des tatsächlichen Bedarfs des Bioreaktors zu erhalten, was zu einer Einsparung von Energie und Verschleiß führt.

**9.** Fermenter/Faulbehälter oder Abwasserspeicher nach Anspruch 2, **dadurch gekennzeichnet, dass** der zwischen den Schaufeln (2) vorgesehene winkelmäßige und axiale Abstand so beschaffen ist, dass die von einer Schaufel (2) erzeugte Wirkung die von der vorhergehenden/nachfolgenden Schaufel (2) erzeugte Wirkung nicht bremst oder beeinflusst.

**10.** Fermenter/Faulbehälter oder Abwasserspeicher nach Anspruch 1, **dadurch gekennzeichnet, dass** die Länge und die besondere Form jeder Schaufel (2), die spiralförmig verformt und mit einer Wölbung gekrümmt ist, die der axialen Komponente der Richtung des Verdrängungsstroms der zu behandelnden Flüssigkeit zugewandt ist, so ausgelegt ist, dass sie die zu behandelnde Flüssigkeitsmasse zur Mitte des Bioreaktors hin schiebt und sie zur nächsten Schaufel (2) lenkt und so bewirkt, dass Folgendes auf die Flüssigkeitsmasse einwirkt:

- eine horizontale Drehbewegung innerhalb des Bioreaktors;
- eine Bewegung der horizontalen Verschiebung von der der Peripherie zum Zentrum des Bioreaktors; sowie
- eine Bewegung des Mischens in der vertikalen Richtung, die die leichteren Grundmassen, die tendenziell aufsteigen, mit den schwereren Grundmassen mischt, die dazu neigen zu sinken, um die zu behandelnde Masse homogenisieren; und
- ein Durchbrechen der freien Oberfläche des Fluids, wodurch die Bildung von Oberflächenkrusten und die Ansammlung von suspendierten Stoffen entfernt und verhindert werden.

**11.** Fermenter/Faulbehälter oder Abwasserspeicher nach einem der vorhergehenden Ansprüche, **dadurch gekennzeichnet, dass** jede Schaufel (2) eine "passive" Reibungsfläche auf den Stützarmen (5) der Schieber (S) vorsieht, die im Vergleich zu bestehenden Schaufeln für langsames Mischen minimiert ist, wobei deren Dicke zum Vorteil einer größeren verfügbaren Energie für den aktiven Schubabschnitt, der durch die Oberfläche der Schieber (S) selbst gebildet wird, reduziert wird.

**Revendications**

**1.** Fermenteur/digesteur ou réservoir de stockage des eaux usées ayant un niveau maximal de remplissage, ledit fermenteur/digesteur ou réservoir de stockage des eaux usées comprenant un mélangeur/agitateur doté d'un arbre rotatif d'axe horizontal (1), ledit arbre (1) étant doté de pales (2), qui sont montées en porte-à-faux et qui ont un développement sensiblement radial par rapport à l'arbre rotatif (1), lesdites pales (2) étant mutuellement échelonnées et étant espacées les unes des autres le long de l'arbre (1) et étant fixées à celui-ci avec un décalage angulaire qui les rend angulairement équidistantes les unes des autres et étant déformées et incurvées de manière à générer un flux de poussée qui est à la fois axial et radial ;

dans le cas où lesdites pales (2) sont configurées pour avoir une longueur telle que, même lorsqu'un fluide à traiter dans lequel elles se déplacent est au niveau maximal de remplissage envisagé par lesdits mélangeurs/agitateurs et réservoirs de stockage des eaux usées, les extrémités libres desdites pales (2) sont configurés pour dépasser ledit niveau maximal de manière à générer un clapotis de la surface libre du fluide à traiter afin d'empêcher toute formation de croûtes de surface ; et

dans lequel lesdites pales (2) ont un grand rayon et un axe de rotation sensiblement horizontal, de manière à générer un mélange uniforme dans lesdits mélangeurs/agitateurs et réservoirs de stockage des eaux usées entre différents gradients de densité, de ce fait en éliminant toute stratification discontinue et en empêchant toute création de zones hétérogènes au niveau de parties inférieures et supérieures desdits mélangeurs/agitateurs et réservoirs de stockage des eaux usées,

dans lequel

lesdites pales (2) ont une forme incurvée en spirale et sont déformées et incurvées dans une direction à l'opposé de la direction de rotation ; dans lequel ledit mélangeur/agitateur envisage une pluralité de pales coaxiales (2), régulièrement espacées le long de l'axe de rotation, avec un décalage de phase angulaire qui les rend angulairement équidistantes les unes des autres.

2. Fermenteur/digesteur ou réservoir de stockage des eaux usées selon la revendication 1, **caractérisé en ce que** ledit mélangeur/agitateur envisage quatre pales coaxiales (2), régulièrement espacées le long de l'axe de rotation, avec un décalage angulaire de 90° les unes des autres.

3. Fermenteur/digesteur ou réservoir de stockage des eaux usées selon l'une quelconque des revendications précédentes, **caractérisé en ce que** lesdites pales (2), qui ont une longueur telle qu'elles émergent légèrement de la surface de la masse fluide à mélanger, sont conçues pour fournir un effet de « rupture de croûtes », provoquant l'immersion forcée d'éventuelles matrices en suspension, en permettant ainsi une recirculation des matrices organiques qui ont tendance à flotter, en les mettant constamment en solution.

4. Fermenteur/digesteur ou réservoir de stockage des eaux usées selon l'une quelconque des revendications précédentes, **caractérisé en ce que** ledit mélangeur/agitateur est constitué d'acier carbone ou d'un autre matériau adapté à cet effet.

5. Fermenteur/digesteur ou réservoir de stockage des eaux usées selon l'une quelconque des revendications précédentes, **caractérisé en ce que** ledit mélangeur/agitateur envisage un premier support (A) à l'intérieur dudit digesteur constitué d'un manchon chromé (3) ajusté sur l'arbre (1) inséré dans un roulement (4) constitué de polyzène antiusure, ainsi qu'un deuxième support (B) en forme de paroi qui contient un roulement à rouleaux et un système à deux joints d'huile pressurisée, pour isoler, dans la mesure du possible, les parties mobiles mécaniques du système du fluide de travail.

6. Fermenteur/digesteur ou réservoir de stockage des eaux usées selon la revendication 5, **caractérisé en ce que** les deux joints extérieurs du deuxième support (B) peuvent être enlevés et remplacés depuis l'extérieur sans avoir besoin d'intervenir dans le fluide à mélanger.

7. Fermenteur/digesteur ou réservoir de stockage des eaux usées selon l'une quelconque des revendications précédentes, **caractérisé en ce que** ledit mélangeur/agitateur est doté de moyens d'entraînement pour permettre un fonctionnement continu, sinon un fonctionnement en mode « pause ».

8. Fermenteur/digesteur ou réservoir de stockage des eaux usées selon la revendication 7, **caractérisé en ce que** ledit mélangeur/agitateur envisage des moyens pour entraîner le moteur pour régler la vitesse de mélange au moyen d'un inverseur destiné à cet effet afin d'obtenir un réglage de la vitesse de mélange sur la base du besoin effectif du bioréacteur, ce qui engendre une économie en termes d'énergie et d'usure.

9. Fermenteur/digesteur ou réservoir de stockage des eaux usées selon la revendication 2, **caractérisé en ce que** la distance angulaire et axiale envisagée entre les pales (2) est telle que le travail généré par une pale (2) ne freine pas et n'affecte pas le travail produit par la pale (2) qui la précède/qui la suit.

10. Fermenteur/digesteur ou réservoir de stockage des eaux usées selon la revendication 1, **caractérisé en ce que** la longueur et la forme particulière de chaque pale (2), qui est déformée en spirale et incurvée avec une concavité faisant face à la composante axiale de la direction du flux de déplacement du liquide à traiter, sont conçues pour pousser, vers le centre du bioréacteur, la masse de liquide à traiter, en la dirigeant vers la pale (2) suivante, et en obtenant ainsi que ce qui suit soit appliqué à ladite masse liquide :

   - un mouvement rotatif horizontal à l'intérieur du bioréacteur ;
   - un mouvement de déplacement horizontal depuis la périphérie vers le centre du bioréacteur ; ainsi que
   - un mouvement de mélange dans la direction verticale qui mélange les matrices plus légères ayant tendance à monter avec les plus lourdes ayant tendance à plonger afin d'homogénéiser la masse à traiter ; et
   - un clapotis de la surface libre du fluide, en éliminant et en empêchant ainsi toute formation de croûtes de surface et d'accumulations de substances en suspension.

11. Fermenteur/digesteur ou réservoir de stockage des eaux usées selon l'une quelconque des revendications précédentes, **caractérisé en ce que** chaque pale (2) envisage une surface de « frottement passif » sur les bras de support (5) des poussoirs (S) qui est minimisée par rapport à des pales existantes à mélange lent, ce qui réduit leur épaisseur avec pour avantage une plus grande énergie disponible pour la section de poussée active constituée par la surface des poussoirs (S) proprement dits.

## PRIOR ART

FIG. 1A

## PRIOR ART

FIG. 1B

**PRIOR ART**

FIG. 1C

**PRIOR ART**

FIG. 1D

## PRIOR ART (SLOW MIXER)

FIG. 1E

## PRIOR ART (SLOW MIXER)

FIG. 1F

12

## PRIOR ART (SLOW MIXER)

FIG. 1G

## PRIOR ART (SLOW MIXER)

FIG. 1H

FIG. 2

FIG. 3

FIG. 4

B

1

FIG. 5

FIG. 6

FIG. 7

S = SECTION OF ACTIVE THRUST

FIG. 8

FIG. 9

FIG. 10

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10260972
- DE 202006004982
- DE 202011052408